# EUROPEAN PATENT SPECIFICATION

(11) **EP 1 653 899 B1**
(45) Date of publication and mention of the grant of the patent: **13.05.2009**
(21) Application number: 04768027.7
(22) Date of filing: 12.08.2004
(51) Int. Cl.: A61F 13/02

(54) **ABSORBENT SHEET WITH LEAKAGE BARRIERS FOR USE IN WOUND DRESSING**
SAUGFÄHIGES FLÄCHENSTÜCK MIT LECKBARRIEREN ZUR VERWENDUNG BEIM VERBINDEN VON WUNDEN
FEUILLE ABSORBANTE ANTI-FUITES POUR PANSEMENT

(30) Priority: 13.08.2003 GB 0319029; 29.09.2003 US 506169 P
(43) Date of publication of application: 10.05.2006
(73) Proprietor: ETHICON, INC., Somerville, NJ 08876 (US)
(72) Inventor: BIOTT, Maurice, Charles, Keighley, West Yorkshire BD20 9LT (GB)
(74) Representative: James, Anthony Christopher W.P.
(86) International application number: PCT/GB2004/003446
(87) International publication number: WO 2005/016197

(56) References cited:
- US-A- 4 015 604
- US-A- 4 762 124
- US-A- 5 681 579
- US-A- 5 968 001
- US-A1- 2003 032 911

## Description

The present invention relates to absorbent sheet materials for use in wound dressings, and to the manufacture of wound dressings containing such materials.

Absorbent dressings are commonly used in medical practice. They frequently comprise an "island" of absorbent sheet material laminated to a central region of a larger, adhesive-coated, semipermeable backing sheet, whereby an adhesive coated margin of the backing sheet extends around the absorbent material for adhering the dressing to the skin of a patient around the wound being treated. In many cases the absorbent island is itself covered by a liquid-permeable wound facing sheet, for example a perforated plastic film sheet or a sheet of liquid-permeable foam or a sheet of hydropolymer. The wound facing sheet may provide a more wound-friendly and non-adherent surface than the absorbent layer material. The wound contacting sheet can be useful to prevent wet-back from the absorbent layer to the wound and/or to prevent excessive drying out of the wound by the absorbent layer, and may provide other advantages.

It has not hitherto been conventional for the wound facing layer of absorbent dressings to be made coterminous with the absorbent layer in such dressings, because of certain problems. A first problem is leakage of wound fluid from the edges of the absorbent layer. Such leakage can soil clothes or bedding. In the case of absorbent island dressings, such leakage can cause loss of adhesion between the adhesive-coated backing sheet and the skin around the wound. Another problem is poor adhesion of the wound contacting sheet to the absorbent layer and/or to the backing sheet when wet, which can result in delamination of the wound facing sheet when the absorbent layer is wet. For these reasons, the wound contacting layer is usually made with a larger area than the absorbent layer so that it extends beyond the absorbent layer and is bonded directly to the adhesive-coated backing sheet around the absorbent layer. In these embodiments, the absorbent layer is in effect received in an envelope defined by the backing sheet and the wound facing sheet. In certain other embodiments, for example as described in GB-A-2348136, the wound contacting sheet is wrapped around the absorbent layer to form an envelope, and then this envelope is adhered to the backing sheet.

US-A-5681579 describes a wound dressing comprising a laminate of an occlusive backing layer a hydrocolloid-containing support layer, and optionally an absorbent layer and an adhesive layer. The laminate may be heat sealed at the edge to reduce leakage of liquid from the edges. A further heat seal for the same purpose may be provided at a location spaced from the edge.

US-A-4762124 describes a liquid dispensing pouch for dispensing cosmetics and medicinal formulations. A support, a liquid impregnated pad, a permeable membrane and a cover layer are peripherally joined in a layered arrangement by adhesive or heat seal means.

US-A-4015604 describes an absorbent sanitary product comprising an absorbent layer in a liquid-permeable envelope. Barrier strips are formed in the absorbent layer to reduce lateral flow of liquid in the absorbent layer.

However, it will be appreciated that the above constructions using a wound contacting layer that is larger than the absorbent sheet entail some additional expense in terms of materials and manufacturing complexity. Relatively large amounts of backing material and wound facing material are needed to achieve a desired liquid absorbency. The problem of delamination of the dressing can still arise due to wound fluid leaking out of the edges of the absorbent layer.

US-A-5968001 describes a wound dressing comprising: an absorbent sheet; a liquid permeable wound facing sheet that is substantially coterminous with the absorbent sheet around all edges thereof; and one or more barrier regions formed in the the absorbent sheet to block lateral transport of liquid within the absorbent sheet to the edges of the absorbent sheet

In a first aspect, the present invention provides a wound dressing comprising: an absorbent sheet; a liquid permeable wound facing sheet that is substantially coterminous with the absorbent sheet around all edges thereof; a liquid impermeable backing layer that is substantially coterminous with the absorbent sheet and the wound facing sheet around all edges thereof; and one or more barrier regions extending around the absorbent sheet to block lateral transport of liquid through the absorbent sheet to the edges of the absorbent sheet, characterised in that the outer edges of the barrier regions are spaced from 1 to 10 mm from the respective edges of the absorbent sheet to define a dry margin around the absorbent sheet, and the absorbent sheet is adhesively bonded to the wound facing sheet by an adhesive applied in said margin between the barrier regions and the edge of the absorbent sheet.

The liquid permeable wound facing sheet is substantially coterminous with the absorbent sheet around all edges thereof. That is to say, the edges of the wound facing sheet preferably substantially coincide with the edges of the absorbent sheet. The wound facing sheet may contact a surface of the absorbent sheet directly, or there may be intermediate layers. A liquid impermeable backing sheet extends over the surface of the absorbent sheet opposite to the wound facing sheet, wherein the liquid permeable backing sheet is also coterminous with the absorbent layer along all edges thereof.

The absorbent sheet may comprise any of the materials conventionally used for absorbing wound fluids, serum or blood in the wound healing art, including gauzes, nonwoven fabrics, hydrophilic foams, freeze-dried or solvent-dried sponges, superabsorbents, hydrogels and mixtures and combinations thereof.

In certain embodiments, the absorbent sheet may be a woven, knitted or nonwoven fabric, for example a nonwoven web of fibers such as viscose staple fibers. The web may optionally contain superabsorbent fibers or particles. In these embodiments, the fabric may comprise thermoplastic fibers such as bicomponent fibers, whereby the liquid blocking region can be formed by application of heat and pressure to form a thermally bonded region of low porosity.

The area of the absorbent sheet is typically in the range of from 1cm² to 200cm², more preferably from 4cm² to 100cm². The basis weight of the absorbent layer may for example be in the range of 50-500g/m², such as 100-400g/m². The uncompressed thickness of the absorbent layer may be in the range of from 0.5mm to 10mm, such as 1mm to 4mm. The free (uncompressed) liquid absorbency measured for physiological saline may be in the range of 5 to 30 g/g at 25°C.

The barrier region normally comprises one or more barrier strips located proximate to an edge of the absorbent sheet. The barrier strips are typically from about 1 mm to about 5mm wide and extend along all sides of the sheet. The outer edge of the barrier region is from 1 to 10 mm from the edge of the absorbent sheet. The barrier regions may be made by any convenient means, but these generally fall into one or more of (a) making the material of the absorbent sheet hydrophobic in the region, for example by applying a hydrophobic finish; and/or (b) reducing the porosity of the absorbent sheet in the barrier region, for example by blocking the pores of the material with a resin such as a silicone adhesive or by permanently compressing the absorbent sheet in the barrier region by thermal, ultrasonic or compressive bonding.

The wound facing layer may be any medically acceptable wound facing sheet, including woven and nonwoven textile materials. In certain embodiments, the wound facing sheet comprises a layer of absorbent foam, such as an open celled hydrophilic polyurethane foam prepared in accordance with EP-A-0541391. In other embodiments, the wound facing layer comprises an apertured thermoplastic film. Suitable thermoplastics include polyolefins such as polyethylene, copolymers such as ethylene methyl acrylate, or fluoropolymers such as polyvinylidene fluoride. In yet other suitable embodiments the wound facing layer comprises an apertured film of water-insoluble hydrogel material. For example, the apertured sheet may be a self-supporting sheet of such a hydrogel material as described in WO03/011352.

For the embodiments wherein the wound facing sheet comprises an apertured film, the apertures typically make up from about 0.1 % to about 50% of the area of the film, more typically from about 1 % to about 30% of the area of the apertured film, and preferably from about 10% to about 25% of the area of the apertured film. Typically, the apertured film has from about 1 to about 30 apertures per square cm, for example from about 4 to about 15 apertures per square cm or from about 5 to about 10 apertures per square cm. In certain embodiments the apertures are uniformly distributed over the surface of the film, preferably in a regular pattern.

The mean area of each aperture may for example be from about 0.01 to about 10 mm², preferably from about 0.1 to about 4 mm², and more preferably from about 1mm² to about 2mm². In certain embodiments, the apertures have a ratio of maximum length to maximum width of from about 1 to about 10, preferably from about 1 to about 3, and more preferably from about 1 to about 1.5. Suitable aperture shapes include round, oval or regular polygonal

The wound facing sheet may be coated on either, or both, surfaces with a layer of hydrogel, which may be apertured, to provide improved control of wound moisture levels and to provide a more wound-friendly dressing. Suitable hydrogel coatings are described in WO02/38097 and WO03/045294.

The wound facing sheet may also consist of, or comprise, or be occlusively coated by, a composition that is soluble in a component of wound exudate, whereby the permeability of the wound facing sheet is dependent on the amount and/or the composition of wound exudate. For example, the composition may be soluble in water to provide increased absorption rates for highly exuding wounds, as described in GB-A-2379392. The composition may be soluble in the presence of enzymes that are markers for wound chronicity, such as protease enzymes, as described in WO03/047643. The composition may be soluble in the presence of enzymes that are markers for wound infection, such as lysozyme, as described in GB-A-2392836. The composition may be soluble in the presence of enzymes that are markers for pain, or other wound conditions. The contents of all of the above applications are hereby incorporated by reference.

It is an advantage of the present invention that an edge of the wound facing sheet coincides with the edge of the absorbent sheet adjacent to the barrier region without adverse consequences, since the barrier region overcomes the problem of liquid leaking out of that edge of the absorbent layer. As already noted, the barrier region extends substantially all around the absorbent layer to block liquid transport to substantially all edges of the absorbent layer. The wound contacting layer is made substantially coterminous with the absorbent layer. Such embodiments are especially easy to make in a continuous process by laminating the wound facing sheet to the absorbent sheet, followed by cutting or die cutting the dressings.

The wound facing sheet is adhesively bonded to the absorbent sheet, by an adhesive applied in a margin between the barrier regions and the edge of the absorbent layer. In certain preferred embodiments, the barrier regions comprise a layer of adhesive extending through the absorbent layer to fix the wound facing layer, the absorbent layer and the backing layer. It is a further advantage of the present invention that the barrier region, by keeping a marginal area of the absorbent sheet dry, prevents loss of adhesion in that region and hence delamination of the wound facing sheet when the absorbent layer is saturated with wound exudates.

The backing sheet is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

The MVTR of the dressing according to the present invention as a whole is lower than that of the backing sheet alone, because the absorbent sheet and wound facing sheet partially obstruct moisture transfer through the dressing. Preferably, the MVTR of the dressing (measured across the island portion of the dressing) is from 20% to 80% of the MVTR of the backing sheet alone, more preferably from 20% to 60% thereof, and most preferably about 40% thereof. It has been found that such moisture vapor transmission rates allow the wound under the dressing to heal under moist conditions without causing the skin surrounding the wound to macerate.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesives useful in the wound dressings of the present invention are suitably moisture vapor transmitting and/or patterned to allow passage of water vapor therethrough. The adhesive is suitably a moisture vapor transmitting, medical grade pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are especially suitable.

It is a further advantage of the present invention that the edges of the backing layer coincide with the edges of the absorbent sheet adjacent to the barrier region, since the barrier region overcomes the problem of liquid leaking out of the dressing to soil clothes or bedclothes. As already noted, the barrier region extends substantially all around the absorbent layer to block liquid transport to substantially all edges of the absorbent layer. The backing layer is substantially coterminous with the absorbent layer and the wound facing layer. Such dressings are referred to as borderless dressings, and may be especially easy to manufacture in a continuous process by simply laminating the absorbent, wound facing, and backing sheets together and then cutting or die-cutting the dressings from the laminate. It will be appreciated that, in such dressings, it may be especially advantageous to have a layer of medically acceptable adhesive on the wound facing side of the wound facing sheet. The adhesive may be coated uniformly on the wound facing surface, or it may be coated around the edges of the wound facing sheet to form an adhesive margin.

The wound facing surface of the dressing is typically protected before use by a removable cover sheet. The cover sheet is normally formed from flexible thermoplastic material or a release-coated paper. Suitable materials include polyesters and polyolefins. The adhesive- facing surface of the cover sheet is usually a release surface. That is to say, a surface that is only weakly adherent to any adhesive on the wound contacting layer. For example, the cover sheet may be formed from a non-adherent plastic such as a fluoropolymer, or it may be provided with a release coating such as a silicone or fluoropolymer release coating.

The wound dressing according to the present invention may be medicated. For example a medicament may be dispersed in the absorbent sheet, or it may be applied to the wound facing sheet, for example in a layer coated on the wound facing sheet. Suitable medicaments include one or more therapeutic agents suitable for the treatment of wounds. In certain embodiments the therapeutic agents are selected from the group consisting of antiseptics, antibiotics, analgesics, steroids, growth factors, and mixtures thereof. Suitable therapeutic agents are the antimicrobials, in particular antibiotics and antiseptics such as colloidal silver, silver sulfadiazine, povidone iodine, chlorhexidine, and mixtures thereof. Suitably, the wound dressing material according to the present invention comprises from about 0.001 mg to about 100mg of the one or more therapeutic substances, more preferably from about 0.05mg to about 20mg.

The wound dressings according to this invention provide at least the following advantages: a) the barrier region bonds and holds the 3 pieces of the dressing together even when wet and b) the barrier region helps to retain and disperse wound fluid within the absorbent layer that would otherwise leak out from the edge of the absorbent layer prior to saturation.

Specific embodiments of the present invention will now be described further, by way of example, with reference to the accompanying drawings, in which:
Figure 1 shows a bottom plan view of a borderless wound dressing according to the invention with the liquid permeable top sheet partially cut away to show the absorbent layer;
Figure 2 shows a schematic cross-sectional view through the dressing of Figure 1;
Figure 3 shows an enlarged detail of Figure 2; and
Figure 4 shows an enlarged detail similar to that of Figure 3, for a further, alternative embodiment of the invention.

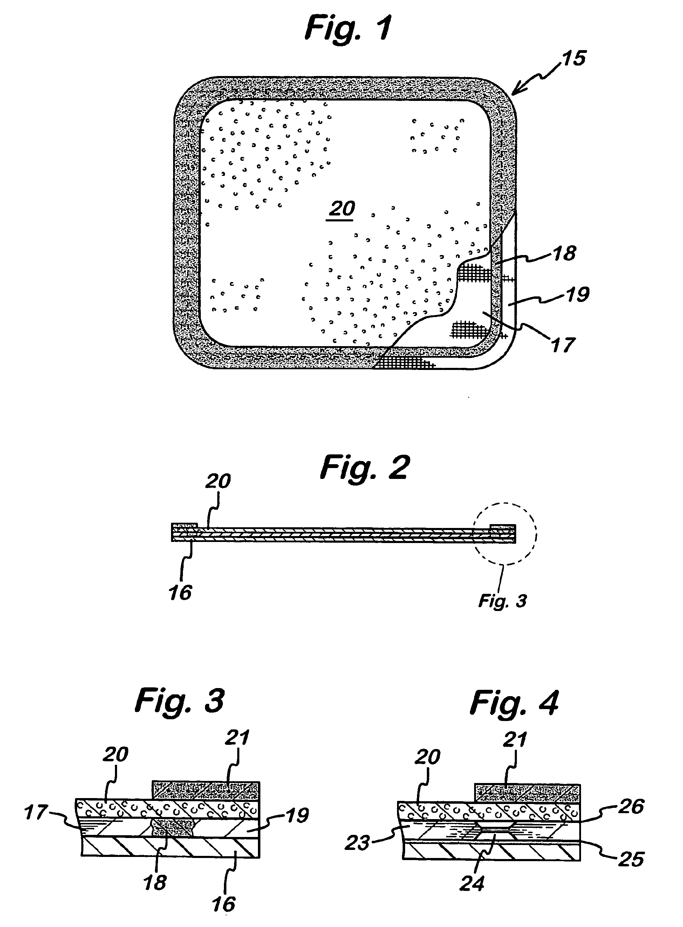

The embodiment shown in Figures 1 to 3 is a so-called borderless dressing in accordance with the present invention. The borderless dressing 15 comprises a microporous, semipermeable polyurethane backing sheet 16 of microporous, semipermeable polyurethane foam coated with a layer of medical-grade, pressure-sensitive polyurethane adhesive.. The borderless dressing 15 further comprises a nonwoven textile absorbent layer 17. The layer 17 is a nonwoven carded web of viscose stable fibers. The textile layer 17 is coterminous with the backing sheet 16.

A barrier strip 18 of width about 3mm (multiple strips could be used) extends around all sides of the absorbent layer 17 a few millimeters from the edge of a dressing. An outer margin 19 of width about 3mm is defined in the absorbent layer by the barrier strips and extends all around the edges of the absorbent layer 17 outside the barrier region. The barrier strip 18 is formed from a hydrophobic silicone adhesive. The barrier strip is produced by impregnating the absorbent sheet 17 in the barrier region with a hydrophobic silicone glue. Alternatively, the barrier regions can be applied directly to the backing sheet. The adhesive gel, or similar, is metered to a suitable nozzle, at a suitable flow rate and applied either to the absorbent layer or to the backing sheet as it travels through the process. Alternatively the absorbent layer, or backing sheet, is held stationary whilst the nozzle, or similar applicator, is moved via an X - Y traverse system.

A wound facing layer 20 of hydrophilic polyurethane foam approximately 1 mm thick, prepared in accordance with EP-A-0541391, extends coterminously with the backing sheet 16 and the absorbent sheet 17.

A margin 21 of medical-grade, pressure-sensitive polyurethane adhesive extends around the inside of the dressing 15 on the wound facing sheet 20. It can be seen from Figure 3 that the adhesive 21 is located above the barrier region 18 and the margin 19 of the absorbent layer. Since these regions remain dry during use of the dressing, the adhesive 21 retains its adherency when the dressing is in use on exuding wounds.

The embodiment shown in partial cross-section in Figure 4 is similar to that of Figures 1 to 3, except that the absorbent sheet 23 includes thermoplastic fibers, and the barrier region 24 has been formed by compressing and heating the absorbent layer 23 to form a compressed and bonded barrier region 24 of low porosity that tends to block the flow of liquid towards the edges of the absorbent layer 23. Since the silicone adhesive 18 of the embodiments of Figures 1 to 3 is no longer present, the embodiment of Figure 4 further comprises optional adhesive layers 25, 26 to bond the absorbent sheet 23 to the polyurethane backing sheet and the polyurethane foam wound facing sheet. Alternatively bonding of 20 to 23 could be a natural bond due to the adhesive nature of the materials used. Fixing of these two components could also be achieved through needling or other methods.

It will be appreciated that the borderless dressing embodiments of Figures 1 to 4 can be manufactured especially easily in continuous processes by laminating the component layers and cutting out the borderless dressings from a continuous strip or sheet.

All of the above-described embodiments normally further comprise a release-coated protective cover sheet or sheets over the wound facing surface and adjacent adhesive surfaces of the dressing, to protect the surfaces prior to use.

The above embodiments have been described by way of example only. Many other embodiments falling within the scope of the accompanying claims will be apparent to the skilled reader.

## Claims

1. A wound dressing (15) comprising:
an absorbent sheet (17);
a liquid permeable wound facing sheet (20) that is substantially coterminous with the absorbent sheet (17) around all edges thereof;
a liquid impermeable backing layer (16) that is substantially coterminous with the absorbent sheet and the wound facing sheet around all edges thereof; and
one or more barrier regions (18) extending around the absorbent sheet (17) to block lateral transport of liquid through the absorbent sheet to the edges of the absorbent sheet, **characterised in that** the outer edges of the barrier regions (18) are spaced from 1 to 10 mm from the respective edges of the absorbent sheet to define a dry margin (19) around the absorbent sheet, and the absorbent sheet is adhesively bonded to the wound facing sheet by an adhesive applied in said margin between the barrier regions (18) and the edge of the absorbent sheet (17) .

2. A wound dressing according to claim 1, wherein the barrier region or regions comprise a hydrophobic material in the absorbent sheet (17) or a region of low porosity in the absorbent sheet (17).

3. A wound dressing according to any preceding claim, wherein the barrier region or regions comprise an adhesive material extending through the absorbent sheet to bond together the absorbent sheet with the wound facing layer and a backing sheet.

4. A wound dressing according to any preceding claim, further comprising a layer of medically acceptable adhesive (21) coated around the edges of the wound facing surface of the wound facing sheet (20) to form an adhesive margin.

## Patentansprüche

1. Wundverband (15), umfassend:
eine absorbierende Schicht (17);
eine flüssigkeitsdurchlässige, zur Wunde gewandte Schicht (20), die im wesentlichen an die absorbierende Schicht (17) entlang deren Kanten angrenzt;
eine flüssigkeitsundurchlässige Gegenschicht (16), die im wesentlichen an die absorbierende Schicht und die zur Wunde gewandte Schicht um deren Kanten angrenzt; und
ein oder mehrere Sperrgebiet(e) (18), das bzw. die sich um die absorbierende Schicht (17) erstreckt bzw. erstrecken, um seitlichen Transport von Flüssigkeit durch die absorbierende Schicht zu den Kanten der absorbierenden Schicht zu blockieren, **dadurch gekennzeichnet, dass** die Außenkanten der Sperrgebiete (18) von 1 bis 10 mn von den jeweiligen Kanten der absorbierenden Schicht beabstandet sind, um einen trockenen Rand (19) um die absorbierende Schicht zu bilden, und die absorbierende Schicht an die zur Wunde gewandte Schicht durch einen Klebstoff, der in den Rand zwischen den Sperrgebieten (18) und der Kante der absorbierenden Schicht (17) aufgetragen ist, geklebt ist.

2. Wundverband nach Anspruch 1, **dadurch gekennzeichnet, dass** das Sperrgebiet bzw. die Sperrgebiete ein hydrophobes Material in der absorbierenden Schicht (17) oder ein Gebiet mit geringer Porosität in der absorbierenden Schicht (18) umfasst bzw. umfassen.

3. Wundverband nach einem der vorangehenden Ansprüche, **dadurch gekennzeichnet, dass** das Sperrgebiet oder die Sperrgebiete ein Klebematerial umfasst bzw. umfassen, das sich durch die absorbierende Schicht erstreckt, um die absorbierende Schicht mit der zur Wunde gewandten Schicht und einer Gegenschicht zu verbinden.

4. Wundverband nach einem der vorangehenden Ansprüche, ferner umfassend eine Schicht aus medizinisch zulässigem Klebstoff (21), die um die Kanten der zur Wunde gewandten Oberfläche der zur Wunde gewandten Schicht (20) durch Beschichtung aufgetragen ist, um einen Kleberand zu bilden.

## Revendications

1. Pansement (15) comprenant :
■ une feuille absorbante (17) ;
■ une feuille perméable au liquide faisant face à la lésion (20) qui est essentiellement coïncidente avec la feuille absorbante (17) tout autour des bords de celle-ci ;
■ une couche support imperméable au liquide (16) qui est essentiellement coïncidente avec la feuille absorbante et la feuille faisant face à la lésion tout autour des bords de celle-ci ; et
■ une ou plusieurs régions barrière (18) entourant la feuille absorbante (17) pour bloquer le transport latéral de liquide à travers la feuille absorbante jusqu'aux bords de la feuille absorbante, **caractérisées en ce que** les bords externes des régions barrière (18) sont espacés de 1 à 10 mm des bords respectifs de la feuille absorbante pour définir une marge sèche (19) autour de la feuille absorbante, et la feuille absorbante est liée de façon adhésive à la feuille faisant face à la lésion par le biais d'un adhésif appliqué dans ladite marge comprise entre les régions barrière (18) et le bord de la feuille absorbante (17) .

2. Pansement selon la revendication 1, dans lequel la région ou les régions barrière comprennent un matériau hydrophobe dans la feuille absorbante (17) ou une région à faible porosité dans la feuille absorbante (17).

3. Pansement selon l'une quelconque des revendications précédentes, dans lequel la région ou les régions barrière comprennent un matériau adhésif passant à travers la feuille absorbante pour lier ensemble la feuille absorbante avec la couche faisant face à la lésion et une feuille support.

4. Pansement selon l'une quelconque des revendications précédentes, comprenant en outre une couche d'adhésif médicalement acceptable (21) enduite autour des bords de la surface faisant face à la lésion de la feuille faisant face à la lésion (20) pour former une marge adhésive.
